# EUROPÄISCHE PATENTSCHRIFT

(11) **EP 1 546 732 B1**
(45) Veröffentlichungstag und Bekanntmachung des Hinweises auf die Patenterteilung: **25.07.2007**
(21) Anmeldenummer: 03750484.2
(22) Anmeldetag: 02.09.2003
(51) Int. Cl.: G01N 33/68, A61P 3/10, G01N 33/74

(54) **MODULATION DER INSULINSYNTHESE**
MODULATION OF THE SYNTHESIS OF INSULIN
MODULATION DE LA SYNTHESE D'INSULINE

(30) Priorität: 03.09.2002 DE 10241111
(43) Veröffentlichungstag der Anmeldung: 29.06.2005
(73) Patentinhaber: Ernst-Moritz-Arndt-Universität Greifswald, 17487 Greifswald (DE)
(72) Erfinder: WALTHER, Reinhard, 17498 Neuenkirchen (DE)
(74) Vertreter: UEXKÜLL & STOLBERG
(86) Internationale Anmeldenummer: PCT/EP2003/009757
(87) Internationale Veröffentlichungsnummer: WO 2004/022772

(56) Entgegenhaltungen:
- EP-A- 1 010 433
- WO-A-01/68108
- WO-A-02/062818
- WO-A-02/062951
- WO-A-02/062954
- LOTTMANN H ET AL: "THE TET-ON SYSTEM IN TRANSGENIC MICE: INHIBITION OF THE MOUSE PDX-1 GENE ACTIVITY BY ANTISENSE RNA EXPRESSION IN PANCREATIC BETA-CELLS" JOURNAL OF MOLECULAR MEDICINE, SPRINGER VERLAG, DE, Bd. 79, Nr. 5/6, Juni 2001 (2001-06), Seiten 321-328, XP001176888 ISSN: 0946-2716 in der Anmeldung erwähnt
- WALTHER R ET AL: "GLUCOSE-INDUCED PHOSPHORYLATION OF PDX-1 BY CASEINE KINASE 2 (CK2)" DIABETOLOGIA, BERLIN, DE, Bd. 46, Nr. SUPPL 2, 24. August 2003 (2003-08-24), Seite A175 XP001184138 ISSN: 0012-186X
- MACFARLANE W M ET AL: "THE P38/REACTIVATING KINASE MITOGEN-ACTIVATED PROTEIN KINASE CASCADE MEDIATES THE ACTIVATION OF THE TRANSCRIPTION FACTOR INSULIN UPSTREAM FACTOR 1 AND INSULIN GENE TRANSCRIPTION BY HIGH GLUCOSE IN PANCREATIC BETA-CELLS" JOURNAL OF BIOLOGICAL CHEMISTRY, AMERICAN SOCIETY OF BIOLOGICAL CHEMISTS, BALTIMORE, MD, US, Bd. 272, Nr. 33, 15. August 1997 (1997-08-15), Seiten 20936-20944, XP001176890 ISSN: 0021-9258 -& DATABASE SWISS-PROT [Online] ID: IPF1_HUMAN, 1. Oktober 1996 (1996-10-01) "PDX-1" Database accession no. P52945 XP002271314
- LOZEMAN F J ET AL: "ISOLATION AND CHARACTERIZATION OF HUMAN CDNA CLONES ENCODING THE ALPHA AND THE ALPHA' SUBUNITS OF CASEIN KINASE II" BIOCHEMISTRY, AMERICAN CHEMICAL SOCIETY. EASTON, PA, US, Bd. 29, Nr. 36, 1990, Seiten 8436-8447, XP001184136 ISSN: 0006-2960 in der Anmeldung erwähnt -& DATABASE SWISS-PROT [Online] ID: KC21_HUMAN, 1. November 1990 (1990-11-01) "Casein kinase II, alpha chain" Database accession no. P19138 XP002271315 -& DATABASE SWISS-PROT [Online] ID: KC22_HUMAN, 1. Februar 1991 (1991-02-01) "Casein kinase II, alpha' chain" Database accession no. P19784 XP002271316
- EHRINGER M A ET AL: "HIGH-THROUGHPUT SEQUENCE IDENTIFICATION OF GENE CODING VARIANTS WITHIN ALCOHOL-RELATED QTLS" MAMMALIAN GENOME, NEW YORK, NY, US, Bd. 12, Nr. 8, 2001, Seiten 657-663, XP001165658 ISSN: 0938-8990 -& DATABASE SWISS-PROT [Online] ID: 143E_HUMAN, 1. November 1995 (1995-11-01) Database accession no. P42655 XP002271318
- RIETZLER M ET AL: "The human WD repeat protein WAIT-1 specifically interacts with the cytoplasmic tails of beta7-integrins." THE JOURNAL OF BIOLOGICAL CHEMISTRY. UNITED STATES 16 OCT 1998, Bd. 273, Nr. 42, 16. Oktober 1998 (1998-10-16), Seiten 27459-27466, XP001188256 ISSN: 0021-9258 -& DATABASE EMBL [Online] 1. Mai 2000 (2000-05-01) "WAIT-1" Database accession no. Q9UNY7 XP002271319

## Beschreibung

Die vorliegende Erfindung betrifft insbesondere die Verwendung von Substanzen, die die Aktivität der Proteine Caseinkinase II (CK II), 14-3-3 epsilon und/oder des PcG-Proteins EED modulieren oder die Bindung der Proteine Caseinkinase II (CK II), 14-3-3 epsilon, des PcG-Proteins EED und/oder eines Fragments derselben mit dem Protein Pancreatic duodenal homeobox-1 (PDX-1), das bei der Glucose-induzierten Insulin-Biosynthese eine entscheidende Rolle spielt, beeinflussen, zur Beeinflussung der Insulin-Synthese bzw. - Bereitstellung.

Im Säugerorganismus wird nach einer Mahlzeit, ausgelöst durch die Glucosebelastung, von den Beta-Zellen der Langerhans'schen Inseln des endokrinen Pancreas in Sekretgranula gespeichertes Insulin sezerniert. Gleichzeitig kommt es zu einer Neusynthese von Insulin (Transcription, Translation). Es wurde festgestellt, daß PDX-1 als Transcriptionsfaktor beteiligt ist (McKinnon and Docherty, Diabetologia (2001), 44: 1203-1214) und die Transkriptions-auslösenden Signalwege durch Wortmannin und SB 203580 hemmbar sind. WO 01/68108 offenbart ein Verfahren zur Induktion der Insulinexpression in kultivierten endokrinen Pankreaszellen, bei dem die Zellen PDX-1 exprimieren und mit einem GLP-1 Rezeptoragonisten behandelt werden. Derzeit ist aber unbekannt, wie die Vorgänge, die zur Sekretion von Insulin führen, und die Induktion der Neusynthese gekoppelt sind.

Aufgabe ist es daher, Substanzen (Wirkstoffe) zur Verfügung zu stellen, die die Insulin-Bereitstellung wirksam beeinflussen und damit zur Behandlung von Erkrankungen geeignet sind, die durch eine verminderte Insulin-Synthese charakterisiert sind oder mit dieser einhergehen, wie z.B. Diabetes.

Im Rahmen der vorliegenden Erfindung wurden überraschend drei Proteine identifiziert, die an den Transkriptionsfaktor PDX-1 (SEQ ID NO: 2) binden, der bei der Glucose-induzierten Insulin-Biosynthese eine entscheidende Rolle spielt (vgl. u.a. Lottmann et al., Journal of Molecular Medicine (2001) 79:321-328). Direkte, unmittelbare Aktivatoren von PDX-1 sind im Stand der Technik bislang nicht bekannt.

Erfindungsgemäß wurden in einem eigens entwickelten experimentellen Zellsystem nach Induktion mit Glucose Proteine identifiziert, die in dieser Phase i) selbst phosphoryliert werden und ii) mit dem Transkriptionsfaktor PDX-1 physikalisch interagieren. PDX-1 selbst wird ebenfalls Glucoseinduziert phosphoryliert, wobei wichtig ist, dass bakteriell exprimiertes PDX-1 erst nach Phosphorylierung an die DNA binden und als Aktivator agieren kann.

In diesem Zusammenhang stellt CK II (SEQ ID NO: 8) in der Insulin-produzierenden Zelle eine Glucose-induzierte PDX-1-Kinase dar. Bei der Caseinkinase II handelt es sich um eine weit verbreitete Serin/Threonin-Kinase. Das Holoenzym besteht einem Tetramer aus zwei alpha- oder alpha'-Untereinheiten (oder jeweils eine dieser Untereinheiten) und zwei beta-Untereinheiten (Lotzeman et al., Biochemistry 36 (1990) 8436-47). Damit kann erfindungsgemäß über die Veränderung der Aktivität dieses Enzyms die Insulin-Bereitstellung moduliert werden. Die Rolle der CK II und Verfahren zu ihrer Modulation durch antisense-Technik sind in WO 02/062954 beschrieben.

Die 14-3-3 Proteine werden als Regulator-Proteine beschrieben, die in der Zelle Schlüsselelemente von Signaltranduktionswegen binden (wie z.B. den Transkriptionsfaktor FKHR) und damit inaktivieren können. Erst durch Phosphorylierung der 14-3-3 Proteine wird diese Bindung aufgehoben. Das Protein 14-3-3 epsilon (SEQ ID NO: 10) hält im unphosphorylierten Zustand den Transkriptionsfaktor PDX-1 gebunden und inaktiv. Nach Glucose-Induktion wird 14-3-3 epsilon phosphoryliert und kann den gebundenen, inaktiven Transkriptionsfaktor PDX-1 freisetzen, der dann als aktivierender Faktor die Insulinsynthese initiiert.

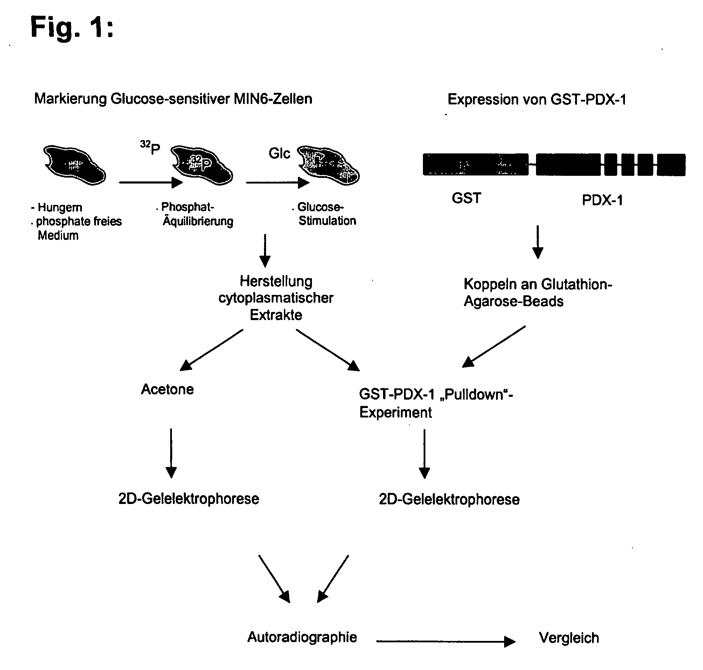

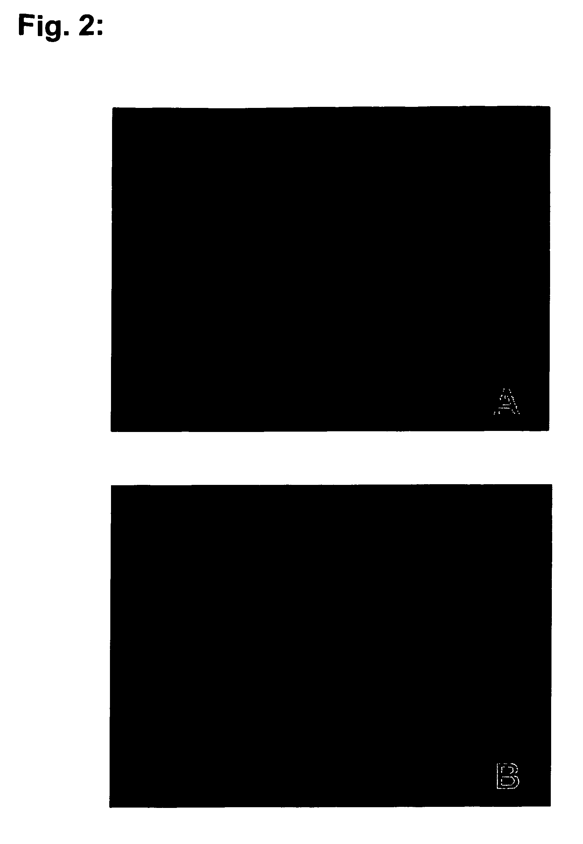

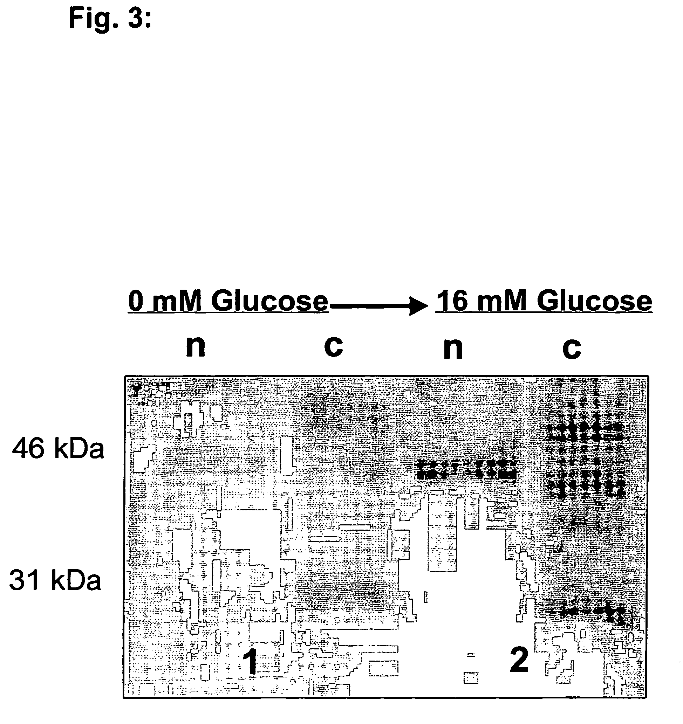

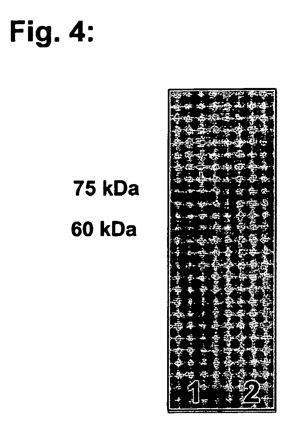

Das EED-Protein (SEQ ID NO: 12) gehört zu den Transkriptions-Repressoren, wobei an dem Vorgang der Repression Histon-Deacetylasen beteiligt zu sein scheinen. Erfindungsgemäß handelt es sich bei der EED um eine große Isoform des Proteins, auf die in Sewalt et al., Mol. Cell. Biol. 18 (1998) 3586-95 (vgl. Fig. 4) verwiesen wird.

Mit den vorliegenden Arbeiten ist es somit gelungen, die drei genannten Proteine (CK II, 14-3-3 epsilon und EED) als wesentliche regulative Elemente der Glucose-induzierten Insulinbiosynthese zu identifizieren. Der oben dargestellte Zusammenhang mit dem Transkriptionsfaktor PDX-1, mit dem die Proteine physikalisch interagieren, ermöglicht nunmehr die Identifizierung neuer Wirkstoffe, die effizient die Insulin-Bereitstellung beeinflussen können und zur Entwicklung einer neuen, effektiveren Generation von Diabetes-Therapeutika mit weniger Nebenwirkungen führen. Das Screening kann mit geeigneten Assays durchgeführt werden, wie z.B. Bindungsassays, mit deren Hilfe sich die Beeinflussung der Wechselwirkung (Bindung) der genannten Proteine mit PDX-1 direkt untersuchen läßt, oder einem Assay, bei dem man die Funktionalisierung von PDX-1 (Phosphorylierung, DNA-Bindung, Transkriptionsaktivierung) unter Wirkstoffeinfluss analysiert. Die Etablierung solcher Assays ist dem Fachmann wohlbekannt

Gegenstand der vorliegenden Erfindung ist daher die Verwendung eines oder mehrerer Proteine gemäß SEQ ID NO: 4 und/oder SEQ ID NO: 6 und SEQ ID NO: 8, 10 und 12 oder Fragmenten derselben zur Durchführung von Bindungsassays unter Verwendung eines Proteins gemäß SEQ ID NQ: 2, wobei die Fragmente an das Protein gemäß SEQ ID NO: 2 binden, zur Identifikation von Substanzen, die die Bindung zwischen dem oder den Proteinen oder Fragment(en) und dem Protein gemäß SEQ ID NO: 2 beeinflussen:

Die Erfindung betrifft unter anderem ein Verfahren zur Identifizierung von Substanzen, die geeignet sind, die Wechselwirkung eines Proteins gemäß SEQ ID NO:4 und/oder SEQ ID NO:6 und SEQ ID NO:8, 10, 12 oder eines Fragments desselben mit dem Protein gemäß SEQ ID NO:2 zu beeinflussen, bei dem man
a) das Protein gemäß SEQ ID NO:4 und/oder SEQ ID NO:6 und SEQ ID NO:8, 10, 12 oder ein Fragment derselben markiert,
b) das Protein gemäß SEQ ID NO:2 markiert,
c) die markierten Proteine von Stufe a) und Stufe b) miteinander in Kontakt bringt und eine Messung zur Bestimmung des/der Markersignals/Markersignale durchführt,
wobei die Markierungen so gewählt sind, daß eine Wechselwirkung der markierten Proteine von Stufe a) und b) nachweisbar und von den isolierten, markierten Proteinen durch Änderung des/der Detekdonssignals/Detektionssignale unterscheidbar ist, man
d) die Mischung von Stufe c) mit einer zu untersuchenden Substanz in Kontakt bringt und man
e) eine weitere Messung zur Bestimmung des/der Markersignals/Markersignale durchführt,
wobei die zu untersuchende Substanz eine die Wechselwirkung beeinflussende Substanz ist, wenn sich das(die) in Stufe e) gemessene(n) Markersignal(e) von dem (den) in Stufe c) gemessenen Markersignal(en) unterscheidet.

Ferner eingeschlossen ist ein Verfahren zur Identifizierung von Substanzen, die geeignet sind, die Wechselwirkung eines Proteins gemäß SEQ ID NO:4 und/oder SEQ ID NO:6 und SEQ ID NO:8, 10, 12 oder eines Fragments derselben mit dem Protein gemäß SEQ ID NO:2 zu beeinflussen, bei dem man entweder
a) das Protein gemäß SEQ ID NO:4 und/oder SEQ ID NO:6 und SEQ ID NO:8 10, 12, ein Fragment derselben oder
b) das Protein gemäß SEQ ID NO:2 auf einer Mikrotiterplatte immobilisiert,
c) das jeweils andere Protein markiert und es mit dem immobilisierten Protein in Kontakt bringt, wobei man das Vorliegen einer Wechselwirkung zwischen den in a) und b) genannten Proteinen nach Durchführung entsprechender Waschschritte durch Nachweis der Markierung bestätigt, man
d) die Proteine mit der zu untersuchenden Substanz in Kontakt bringt,
wobei die zu untersuchende Substanz eine die Wechselwirkung beeinflussende Substanz ist, wenn die Markierung nach Zugabe der zu untersuchenden Substanz und Durchführung entsprechender Waschschritte auf den Mikrotiterplatten nicht mehr nachweisbar ist.

Die mit Hilfe der durchgeführten Screening-Verfahren identifizierten Wirkstoffe können zur Behandlung (patho)physiologischer Zustände, bei denen eine gegenüber dem Normalwert verminderte Insulinproduktion beobachtet wird, eingesetzt werden.

Vorliegend werden unter einer Erkrankung, die durch eine verminderte Insulin-Synthese charakterisiert ist oder mit einer verminderten Insulin-Synthese einhergeht, verschiedene Formen von Diabetes, wie z.B. Diabetes mellitus verstanden.

Gemäß einer besonderen Ausführungsform können auch eines oder mehrere Proteine gemäß SEQ ID NO:4 und/oder SEQ ID NO:6 und SEQ ID NO:8, 10 und 12 zur Herstellung eines pharmazeutischen Präparates zur Behandlung einer Erkrankung, die durch eine verminderte Insulin-Synthese charakterisiert ist oder mit dieser einhergeht, verwendet werden.

Die Erfindung betrifft ferner die Verwendung einer oder mehrerer Nukleinsäuren gemäß SEQ ID NO: 3 und/oder 5 und 7 oder 9 und/oder einer oder mehrerer für EED kodierender Nukleinsäuren zur Herstellung eines pharmazeutischen Präparates zur Behandlung einer Erkrankung, die durch eine verminderte Insulin-Synthese charakterisiert ist oder mit dieser einhergeht. Diese Präparate können beispielsweise in der Gentherapie, z.B. bei der Generierung artifizieller, insulinproduzierender Zellen für eine Transplantation, Anwendung finden.

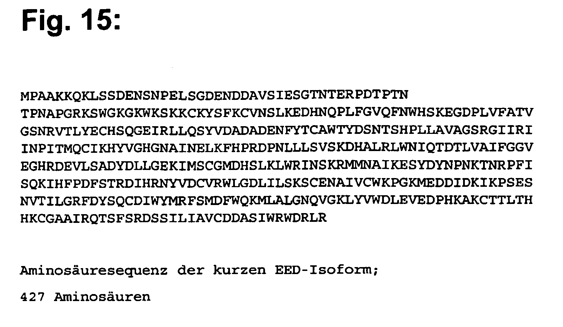

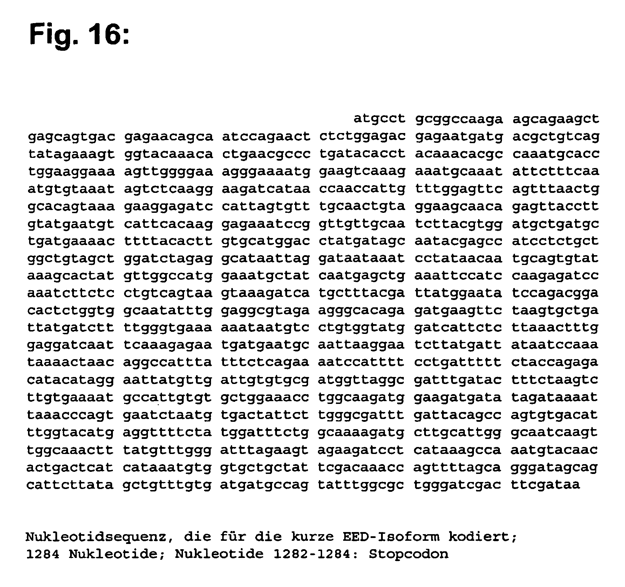

Im Rahmen der durchgeführten Arbeiten wurde erstmals das Protein (EED), bei dem es sich um eine EED-Isoform handelt (vgl. Fig. 4 in Sewalt et al. Mol Cell Biol (1998), 18(6): 3586-95), als regulatives Element identifiziert, das ebenfalls eine bedeutende Rolle bei der Insulinbiosynthese spielt. Erfindungsgemäß eingeschlossen sind Fragmente der vorgenannten Proteine, wobei der Begriff EED auch die kürzere Isoformen von EED einschließt (vgl. Fig. 15).

Anhand der gewonnenen Erkenntnisse lassen sich Assays zur Messung der Funktionalisierung von PDX-1 konstruieren, die Aufschluss über die Eigenschaft von Substanzen geben, die Bindung von PDX-1 an den Promotor des Insulin-Gens zu inhibieren bzw. zu fördern. So lassen sich anhand von molekularbiologischen Standardverfahren beispielsweise transgene Zellkulturen etablieren, in die man ein Reportergen einbringt, dessen Genprodukt leicht detektierbar und quantifizierbar ist, und das unter der Kontrolle eines Promotors mit PDX-1-bindenden DNA-Sequenzen stabil exprimiert wird. Unter Induktionsbedingungen, d.h. bei Bindung von PDX-1 an den Promotor, kann dann die Expression des Reportergens unter Einfluss der zu testenden Substanz analysiert werden.

Die Erfindung wird nachfolgend anhand von Beispielen erläutert.

### Beispiele

### Material und Methoden

### Identifizierung von Glucose-induzierten phosphylierten Interaktionspartnern von PDX-1:

MIN 6-Zellen wurden bis zur 80%-igen Konfluenz in DMEM kultiviert, das 25 mM Glucose, 10% Pferde-Serum und 2,5% FCS (fötales Kälber-Serum) enthielt. Die Zellen wurden zwei Mal gewaschen, und man ließ die Zellen im Krebs-Ringer-Puffer (118 mM Natriumchlorid, 4,75 mM Kaliumchlorid, 1,25 mM Kalziumchlorid, 1,2 mM Magnesiumchlorid, 0,05% (w/v) BSA, 25 mM Natriumhydrogencarbonat, 10 mM Hepes, pH 7,4) für drei Stunden hungern. Nach der dreistündigen Vorinkubation der Zellen wurden diese mit 500 µCi/ml (³² P)-Phosphorsäure (ICN) für eine Stunde equilibriert und anschließend in Gegenwart oder Abwesenheit von 16 mM Glucose mit und ohne die Kinaseinhibitoren Wortmannin (100 nM, 10 min Vorinkubation) und SB203580 [(4-(4-Fluorphenyl)-2-(4-methylsulfinylphenyl)-5-(4-pyridyl)-imidazol] (10 mM, 30 min Vorinkubation) für weitere 30 min inkubiert. Anschließend wurden die Zellen mit eiskaltem PBS gewaschen und geerntet. Die Zellen wurden für 30 Sekunden zentrifugiert und in 100 µl 20 mM Hepes 7,8, 50 mM Kaliumchlorid, 1% Triton X-100, 0,1 mM EDTA, 20 mM β-Glycerophosphat, 0,1 mM Na₃PO₄, vollständiger Miniprotease Inhibitorcocktail (Roche), resuspendiert. Die Zellen ließ man für 10 Minuten auf Eis anschwellen und zentrifugierte anschließend bei 14.000 U/min. Der Überstand (cytoplasmatischer Extrakt) wurde in ein frisches Eppendorf-Gefäß überführt, und die Proteine wurden unter der Verwendung von Aceton präzipitiert, mehrfach in Aceton gewaschen, getrocknet und in Lysis-Puffer (8 M Harnstoff, 2 M Thioharnstoff, 65 mM Chaps, 120 mM DTT, 80 mM Tris) solubilisiert. Für die präparativen Gele wurden die markierten Extrakte mit 4 mg nicht-radioaktiven cytoplasmatischen Extrakten gemischt, die ähnlich behandelt wurden. Die Proteintrennung erfolgte unter Verwendung eines immobilisierten pH-Gradienten von 3 bis 10 (IPG-Streifen, 18 cm, Amersham Biosciences) in einen IPGphor-isoelektrischem-Focussier-System (Amersham Biosciences). Nach Equilibrieren des Streifens in SDS-Puffer wurde die zweite Dimension der SDS-PAGE über Nacht unter Verwendung eines 12,5%igen Acrylamidgels durchgeführt. Die präparativen Gele wurden mit kolloidalem Coomassi R-250 gefärbt und einer Autoradiographie unter Verwendung eines Phosphor-Imagers (Molecular dynamics) unterworfen. Die analytischen Gele wurden einer Autoradiographie unterworfen. Es wurden sogenannte "Pulldown-Experimente" unter Verwendung eines bakteriellen exprimierten GST-PDX-1 Proteins als Köder im Cytoplasma von Glucose stimulierten, ³²P-markierten MIN6 Zellen durchgeführt. Nach Zugabe von 5 µg GST zur Absättigung unspezifischer Bindungsstellen wurde GST-PDX-1-Fusionsprotein, das an Gluthation-Agarose-Beads gekoppelt war, mit 100 mg cytoplasmatischem Extrakt bei drei Stunden und 4°C und unter durchgehendem Schütteln inkubiert. Nach dieser Inkubationsphase wurden die Beads bei 3000 g für 5 Minuten pelletiert, und der Überstand wurde gesammelt und für die zweidimensionale Gelelektrophorese verwendet. Um die Interaktion von Phosphoproteinen mit dem GST-PDX-1 Protein nachzuweisen, wurden die pelletierten Beads 3 Mal gewaschen, in Lysis-Puffer suspendiert und anschließend der zweidimensionalen Gelelektrophorese unterworfen. Die Immunfluoreszenz-Färbung und das Western-Blotting wurden unter Verwendung von Standard-molekularbiologischen Protokollen verwendet.

### Ergebnisse:

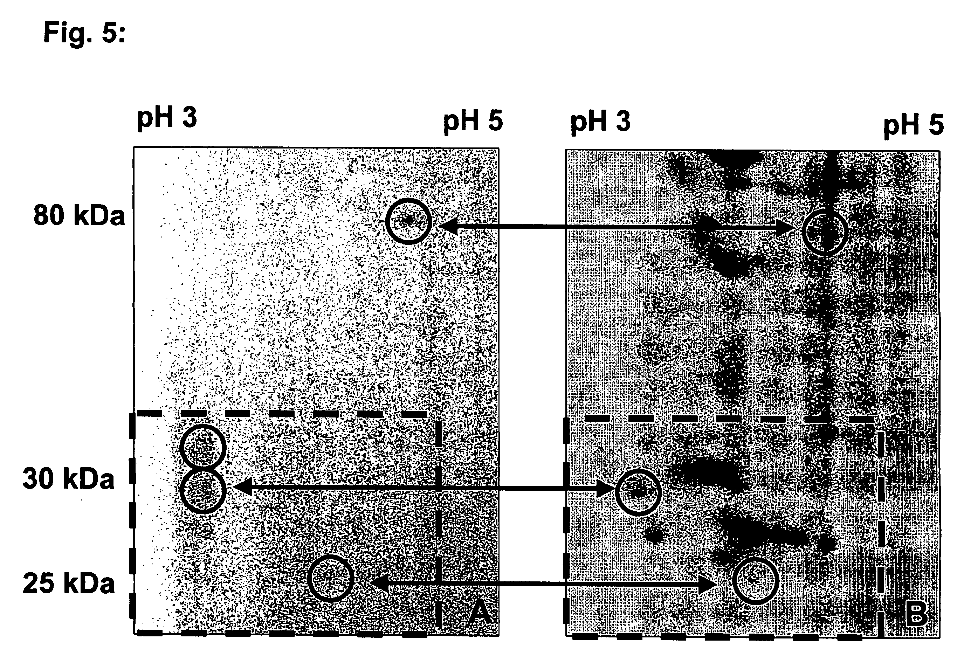

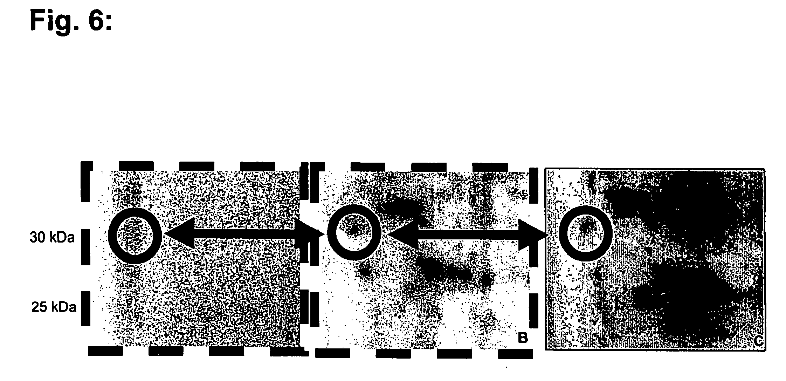

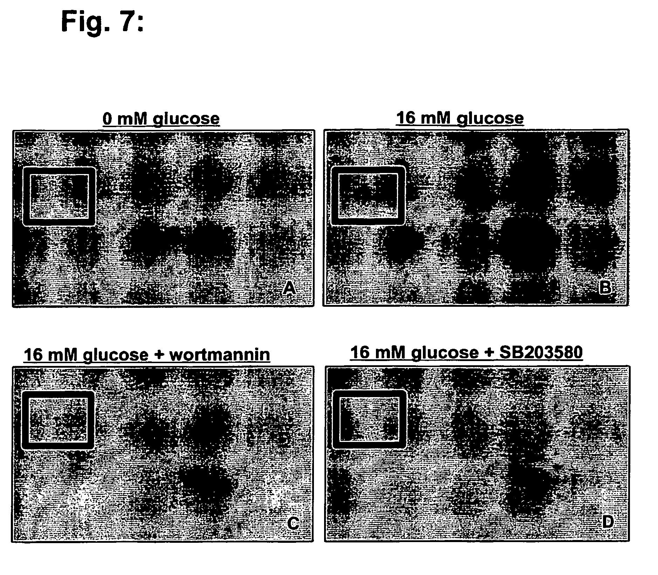

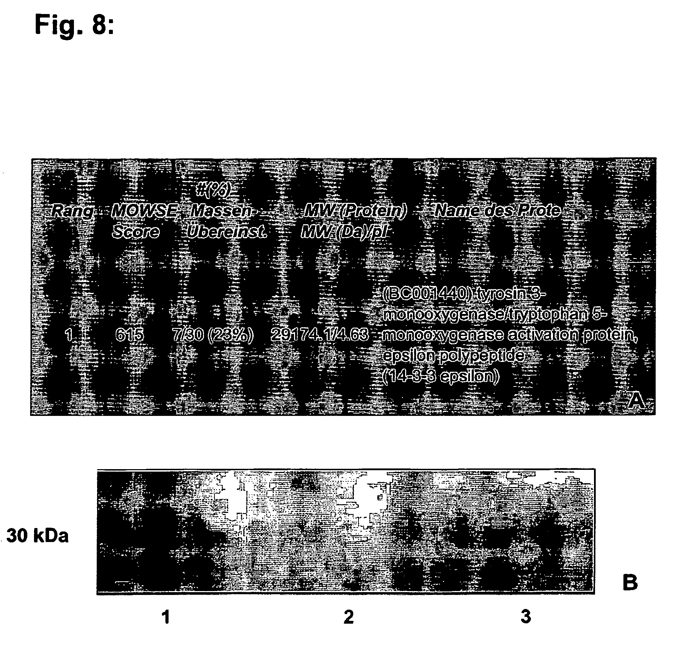

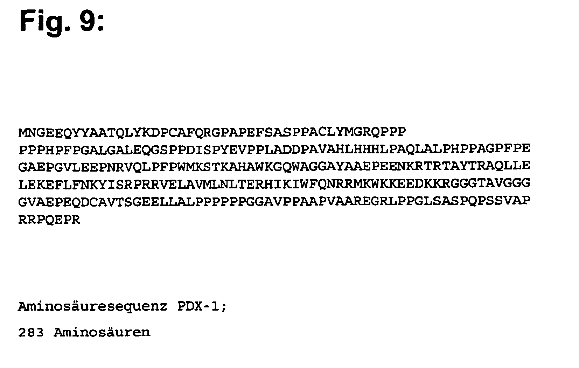

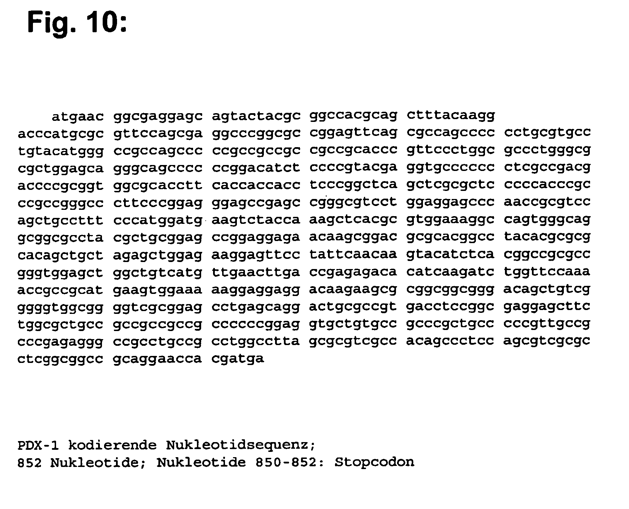

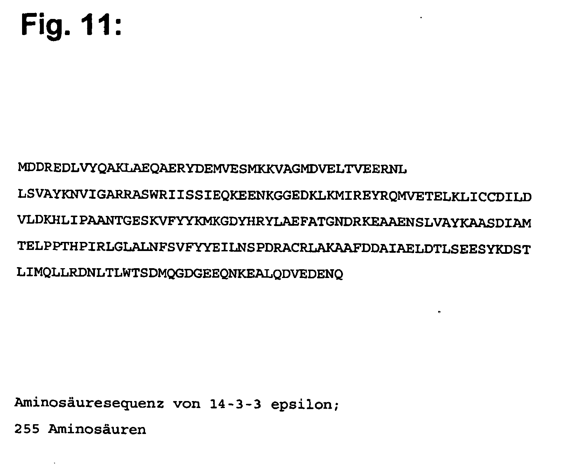

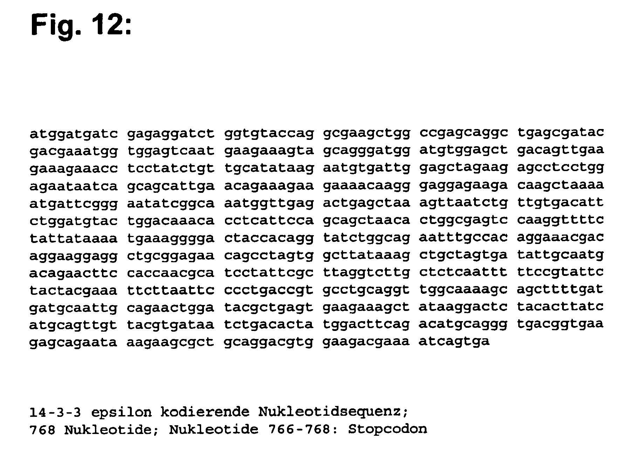

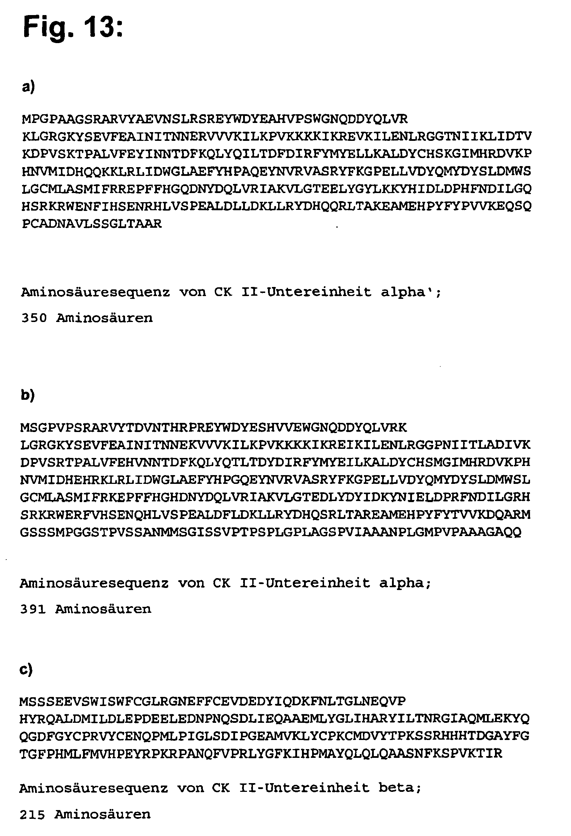

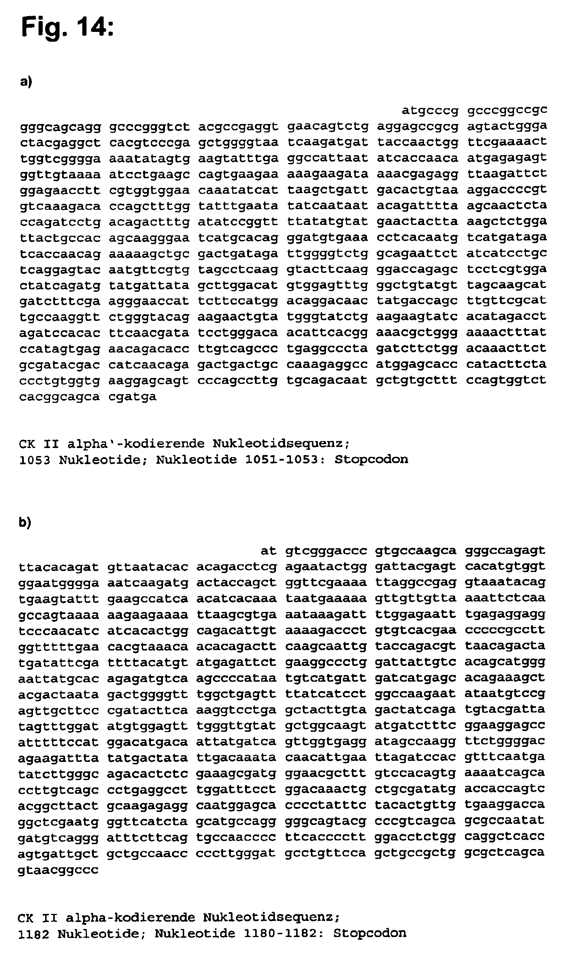

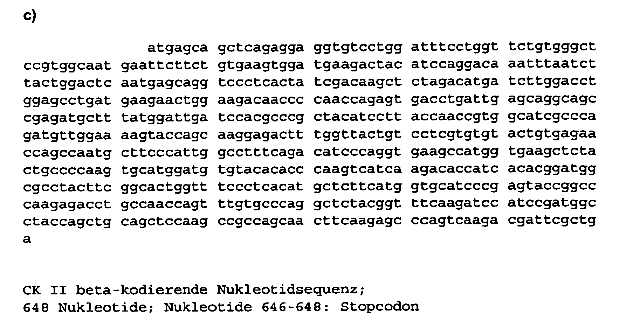

Im Autoradiogramm des analytischen Geles des "Pulldown" (Fig 5, A) wurden Spots von phosphorylierten, mit PDX-1-interagierenden Proteinen detektiert. Diese Spots konnten in präparativen Gelen Coomassie-gefäbten Spots zugeordnet werden und wurden mittels Massenspektrometrie (MALDI-TOF) identifiziert.

### Beschreibung der Figuren

Figur 1:
   Schematische Darstellung des Nachweises von Glucose-induzierten Interaktionspartnern von PDX-1
Figur 2:
   Auswirkung von Glucose auf die subzelluläre Lokalisation von endogenem PDX-1. A: Die Zellen wurden in Krebs-Ringer-Puffer für vier Stunden mit 0 mM Glucose inkubiert. B: Die Kultur wurde anschließend 30 Minuten bei 16 mM Glucose weiterkultiviert. Endogenes PDX-1 wurde unter Verwendung eines polyclonalen Anti-PDX-1 Antiserums nachgewiesen.
Figur 3:
   PDX-1 wird bei hohen Glucosekonzentrationen in MIN6-Zellen modifiziert. Westem-Blot-Analyse von nukleären und cytoplasmatischen Extrakten, die aus MIN6-Zellen hergestellt wurden, und in Krebs-Ringer-Puffer bei 0 mM Glucose (Bahn 1) inkubiert und anschließend auf 16 mM Glucose (Bahn 2) transferiert wurden.
Figur 4:
   Silberfärbung von bakteriell exprimiertem GST-PDX-1. 5 µg (Bahn 1) und 1 µg (Bahn 2) gereinigtes GST-PDX-1; in E. coli exprimiert und mittels Polyacrylamidgelelektrophorese getrennt.
Figur 5:
   Bakteriell exprimiertes GST-PDX-1 präzipitiert Phosphoproteine aus dem Cytoplasma von ³²P-markierten, Glucose-stimulierten MIN6-Zellen.
   A: Kartierung von erhaltenen Phosphoproteinen, die durch zweidimensionale Gelelektrophorese nach einem GST-PDX-1-Pulldown-Experiment erhalten wurden.
   B: Cytoplasmatische Phosphoproteine, die durch zweidimensionale Gelelektrophorese nach Acetonpräzipitation getrennt wurden.
Figur 6:
   Bakteriell exprimiertes GST-PDX-1 verringert die Menge eines Phosphoproteins im Überstand von ³²P-markierten, Glucose-stimulierten MIN6-Zellen vor der zweidimensionalen Gelelektrophorese.
   A: Die vergrößerte Region des 2D-Gels, das einer Autoradiographie wurde, zeigt die erhaltenen Phosphoproteine.
   B: Die vergrößerte Region eines 2D-Gels, das der Autoradiographie unterworfen wurde, zeigt cytoplasmatische Phosphoproteine nach Acetonpräzipitation.
   C: Die vergrößerte Region eines 2D-Gels, das der Autoradiographie unterworfen wurde, zeigt cytoplasmatische Phosphoproteine nach Acetonpräcipitation mit nachfolgender Inkubation mit GST-PDX-1 Fusionsproteinen.
Figur 7:
   Veränderungen des Phosphorylierungszustands des ausgewählten cytoplasmatischen Proteins als Reaktion auf Glucose und verschiedene Kinaseinhibitoren.
A-D:
   Vergrößerte Regionen von 2D-Gelen, die der Autoradiographie nach erfolgter Acetonpräzipitation unterworfen wurden.
   A: MIN6-Zellen wurden für 8 Stunden bei 0 mM Glucose in Krebs-Ringer-Puffer inkubiert.
   B: Die Kultur wurde auf 16 mM Glucose transferiert.
   C: Die Kultur wurde auf 16 mM Glucose in Gegenwart von Wortmannin (100 nM) transferiert.
   D: Die Kultur wurde auf 16 mM Glucose in Gegenwart von SB203580 (10 µM) transferiert.
Figur 8:
   Identifizierung von 14-3-3-epsilon als Interaktionspartner von GST-PDX-1.
   A: Die Ergebnisse der MS-Fit-Suche ergaben Massen eines Phosphoproteins, welches quantitativ aus dem Cytoplasma von Glucose-behandelten MIN6-Zellen unter Verwendung eines bakteriellen exprimierten GST-PDX-1 Proteins präzipitiert werden konnte.
   B: Western-Blot unter Verwendung eines Anti-1-4-3-Epsilon-Antiserums (Santa Cruz). Bahn 1 (Positivkontrolle): 30 µg cytoplasmatischer Extrakt von Glucose-behandelten MIN6-Zellen. Bahn 2 (Negativkontrolle): GST-Pulldown von 400 µg Glucose-behandelten MIN6-Zellen. Bahn 3: GST-PDX-1 Pulldown von 400 µg Glucose-behandelten MIN6-Zellen.
Figur 9:
   Aminosäuresequenz von PDX-1 (SEQ ID NO:2).
Figur 10:
   Für PDX-1 kodierende Nukleotidsequenz (SEQ ID NO:1).
Figur 11:
   Aminosäuresequenz von 14-3-3 epsilon (SEQ ID NO:10).
Figur 12:
   Für 14-3-3 epsilon kodierende Nukleotidsequenz (SEQ ID NO:9).
Figur 13:
   Aminosäuresequenz der CK II-Untereinheiten
   a) Aminosäuresequenz von CKII-alpha' (SEQ ID NO:4)
   b) Aminosäuresequenz von CKII-alpha (SEQ ID NO:6)
   c) Aminosäuresequenz von CKII-beta (SEQ ID NO:8)
Figur 14:
   Für die CK II-Untereinheiten kodierende Nukleotidsequenz
   a) für CKII-alpha' kodierende Nukleotidsequenz (SEQ ID NO:3)
   b) für CKII-alpha kodierende Nukleotidsequenz (SEQ ID NO:5)
   c) für CKII-beta kodierende Nukleotidsequenz (SEQ ID NO:7)
Figur 15:
   Nukleotidsequenzen der kurzen EED-Isoform (SEQ ID NO:11)
Figur 16:
   Aminosäuresequenzen der kurzen EED-Isoform (SEQ ID NO:12)

## Patentansprüche

1. Verwendung eines oder mehrerer Proteine gemäß SEQ ID NO:4 und/oder SEQ ID NO:6 und SEQ ID NO:8, SEQ ID N0:10, SEQ ID N0:12 oder Fragmenten derselben zur Durchführung von Bindungsassays unter Verwendung eines Proteins gemäß SEQ ID NO:2, wobei die Fragmente an das Protein gemäß SEQ ID NO:2 binden, zur Identifikation von Substanzen, die die Bindung zwischen dem oder den Proteinen oder Fragment(en) und dem Protein gemäß SEQ ID NO:2 beeinflussen.

2. Verfahren zur Identifizierung von Substanzen, die geeignet sind, die Wechselwirkung eines Proteins gemäß SEQ ID NO:4 und/oder SEQ ID NO:6 und SEQ ID NO:8, SEQ ID NO:10, SEQ ID NO:12 oder eines Fragments derselben mit dem Protein gemäß SEQ ID NO:2 zu beeinflussen, wobei das Fragment an das Protein gemäß SEQ ID NO:2 bindet, bei dem man
a) das Protein gemäß SEQ ID NO:4 und/oder SEQ ID NO:6 und SEQ ID NO:8, SEQ ID NO: 10, SEQ ID NO: 12 oder das Fragment derselben markiert,
b) das Protein gemäß SEQ ID NO:2 markiert,
c) die markierten Proteine von Stufe a) und Stufe b) miteinander in Kontakt bringt und eine Messung zur Bestimmung des/der Markersignals/Markersignale durchführt,
wobei die Markierungen so gewählt sind, daß eine Wechselwirkung der markierten Proteine von Stufe a) und b) nachweisbar und von den isolierten, markierten Proteinen durch Änderung des/der Detektionssignals/Detektionssignale unterscheidbar ist, man
d) die Mischung von Stufe c) mit einer zu untersuchenden Substanz in Kontakt bringt und man
e) eine weitere Messung zur Bestimmung des/der Markersignals/Markersignale durchführt,
wobei die zu untersuchende Substanz eine die Wechselwirkung beeinflussende Substanz ist, wenn sich das(die) in Stufe e) gemessene(n) Markersignal(e) von dem(den) in Stufe c) gemessenen Markersignal(en) unterscheidet.

3. Verfahren zur Identifizierung von Substanzen, die geeignet sind, die Wechselwirkung eines Proteins gemäß SEQ ID NO:4 und/oder SEQ ID NO:6 und SEQ ID NO:8, SEQ ID NO:10, SEQ ID NO:12 oder eines Fragments derselben mit dem Protein gemäß SEQ ID NO:2 zu beeinflussen, wobei das Fragment an das Protein gemäß SEQ ID NO:2 bindet, bei dem man entweder
a) das Protein gemäß SEQ ID NO:4 und/oder SEQ ID NO:6 und SEQ ID NO:8, SEQ ID NO: 10, SEQ ID NO: 12 oder das Fragment derselben oder
b) das Protein gemäß SEQ ID NO:2 auf einer Mikrotiterplatte immobilisiert,
c) das jeweils andere Protein markiert und es mit dem immobilisierten Protein in Kontakt bringt, wobei man das Vorliegen einer Wechselwirkung zwischen den in a) und b) genannten Proteinen nach Durchführung entsprechender Waschschritte durch Nachweis der Markierung bestätigt, man
d) die Proteine mit der zu untersuchenden Substanz in Kontakt bringt,
wobei die zu untersuchende Substanz eine die Wechselwirkung beeinflussende Substanz ist, wenn die Markierung nach Zugabe der zu untersuchenden Substanz und Durchführung entsprechender Waschschritte auf den Mikrotiterplatten nicht mehr nachweisbar ist.

4. Verwendung eines oder mehrerer Proteine gemäß SEQ ID NO:4 und/oder SEQ ID NO:6 und SEQ ID NO:8, SEQ ID NO:10, SEQ ID NO:12 zur Herstellung eines pharmazeutischen Präparates zur Behandlung einer Erkrankung, die durch eine verminderte Insulin-Synthese charakterisiert ist oder mit dieser einhergeht.

5. Verwendung einer oder mehrerer Nukleinsäuren gemäß SEQ ID NO: 3 und/oder 5 und 7 oder 9 und/oder einer oder mehrerer für das Protein gemäß SEQ ID NO:12 kodierender Nukleinsäuren zur Herstellung eines pharmazeutischen Präparates zur Behandlung einer Erkrankung, die durch eine verminderte Insulin-Synthese charakterisiert ist oder mit dieser einhergeht.

6. Verwendung gemäß den Ansprüchen 4 oder 5, **dadurch gekennzeichnet, daß** die Erkrankung Diabetes ist.

## Claims

1. Use of one or several proteins according to SEQ ID NO:4 and/or SEQ ID NO:6 and SEQ ID NO:8, SEQ ID NO: 10, SEQ ID NO:12 or fragments of said proteins, for performing binding assays using a protein according to SEQ ID NO:2, wherein the fragments bind to the protein according to SEQ ID NO:2, for the identification of substances that influence binding between the protein or proteins or fragment(s) and the protein according to SEQ ID NO:2.

2. A process for identifying substances that are suitable for influencing interaction of a protein according to SEQ ID NO:4 and/or SEQ ID NO:6 and SEQ ID NO:8, SEQ ID NO:10, SEQ ID NO:12 or a fragment of said proteins, with the protein according to SEQ ID NO:2, wherein
a) The protein according to SEQ ID N0:4 and/or SEQ ID N0:6 and SEQ ID NO:8, SEQ ID NO:10, SEQ ID NO:12 or the fragment of said proteins, is labelled,
b) The protein according to SEQ ID NO:2 is labelled,
c) The labelled proteins from step a) and step b) are brought into contact with each other and a measurement is performed to determine the signal or signals of the label(s),
wherein the labels are selected so that interaction of labelled proteins from step a) and b) can be detected and distinguished from the isolated unlabelled proteins via alteration of the detection signal/detection signals,
d) The mixture from step c) is brought into contact with a substance to be examined, and
e) Another measurement is performed to determine the signal or signals of the label(s),
wherein the substance to be examined is a substance that influences the interaction, if the signal(s) of the label(s) measured in step e) differ(s) from the signal(s) of the label(s) measured in step c).

3. A process for identifying substances that are suitable for influencing interaction of a protein according to SEQ ID NO:4 and/or SEQ ID NO:6 and SEQ ID NO:8, SEQ ID NO:10, SEQ ID N0:12 or a fragment of said proteins, with the protein according to SEQ ID NO:2, wherein
a) The protein according to SEQ ID NO:4 and/or SEQ ID NO:6 and SEQ ID NO:8, SEQ ID NO:10, SEQ ID NO:12 or the fragment of said proteins, or
b) The protein according to SEQ ID NO:2 is immobilised on a microtiter plate,
c) The other protein in question is labelled and brought into contact with the immobilised protein, wherein the presence of an interaction between the proteins mentioned in a) and b) is confirmed by detecting the labelling after performing corresponding washing steps,
d) The proteins are brought into contact with the substance to be examined,
wherein the substance to be examined is a substance influencing the interaction if, after addition of the substance to be examined and performing corresponding washing steps on the microtiter plates, the labelling is no longer detectable.

4. The use of one or several proteins according to SEQ ID NO:4 and/or SEQ ID NO:6 and SEQ ID NO:8, SEQ ID NO:10, SEQ ID NO:12 for the manufacture of a pharmaceutical preparation for the treatment of a disease that is **characterised by** decreased synthesis of insulin or that is accompanied by decreased synthesis of insulin.

5. Use of one or several nucleic acids according to SEQ ID NO : 3 and/or 5 and 7 or 9, and/or one or several nucleic acids that encode the protein according to SEQ ID N0:12 for the manufacture of a pharmaceutical preparation for the treatment of a disease that is **characterised by** decreased synthesis of insulin or that is accompanied by decreased synthesis of insulin 1.

6. The use according to claims 4 or 5, wherein the disease is diabetes.

## Revendications

1. Utilisation d'une ou plusieurs protéines selon SEQ ID n° 4 et/ou SEQ ID n° 6 et SEQ ID n° 8, SEQ ID n° 10, SEQ ID n° 12 ou de fragments de celles-ci, pour l'exécution d'un essai de liaison avec utilisation d'une protéine selon SEQ ID n° 2, les fragments se liant à la protéine selon SEQ ID n° 2, pour l'identification de substances qui influent sur la liaison entre la ou les protéines ou le ou les fragments et la protéine selon SEQ ID n° 2.

2. Procédé pour l'identification de substances qui sont appropriées à influer sur l'interaction d'une protéine selon SEQ ID n° 4 et/ou SEQ ID n° 6 et SEQ ID n° 8, SEQ ID n° 10, SEQ ID n° 12 ou d'un fragment de celle-ci avec la protéine selon SEQ ID n° 2, le fragment se liant à la protéine selon SEQ ID n° 2, dans lequel
a) on marque la protéine selon SEQ ID n° 4 et/ou SEQ ID n° 6 et SEQ ID n° 8, SEQ ID n° 10, SEQ ID n° 12 ou le fragment de celle-ci,
b) on marque la protéine selon SEQ ID n° 2,
c) on met en contact entre elles les protéines marquées de l'étape a) et de l'étape b) et on effectue une mesure pour la détermination du signal marqueur/des signaux marqueurs,
les marqueurs étant choisis de manière à pouvoir déceler une interaction des protéines marquées de l'étape a) et de l'étape b) et distinguer les protéines isolées marquées, par modification de ce signal de détection/ces signaux de détection,
d) on met le mélange de l'étape c) en contact avec une substance à étudier et
e) on effectue une autre mesure pour la détermination du signal de détection/des signaux de détection,
la substance à étudier étant une substance influant sur l'interaction lorsque le signal de marqueur/les signaux de marqueurs mesuré(s) dans l'étape e) diffère(nt) du signal de marqueur/des signaux de marqueurs mesuré(s) dans l'étape c).

3. Procédé pour l'identification de substances qui sont appropriées à influer sur l'interaction d'une protéine selon SEQ ID n° 4 et/ou SEQ ID n° 6 et SEQ ID n° 8, SEQ ID n° 10, SEQ ID n° 12 ou d'un fragment de celle-ci avec la protéine selon SEQ ID n° 2, le fragment se liant à la protéine selon SEQ ID n° 2, dans lequel on immobilise sur une plaque de microtitrage soit
a) la protéine selon SEQ ID n° 4 et/ou SEQ ID n° 6 et SEQ ID n° 8, SEQ ID n° 10, SEQ ID n° 12 ou le fragment de celle-ci, soit
b) la protéine selon SEQ ID n° 2,
c) on marque l'autre protéine et on la met en contact avec la protéine immobilisée, de sorte qu'après exécution d'étapes de lavage appropriées on confirme par détection du marquage la présence d'une interaction entre les protéines mentionnées en a) et b),
c) on met les protéines en contact avec la substance à étudier,
la substance à étudier étant une substance influant sur l'interaction lorsque après addition de la substance à étudier et exécution d'étapes de lavage appropriées, le marquage n'est plus détectable sur les plaques de microtitrage.

4. Utilisation d'une ou plusieurs protéines selon SEQ ID n° 4 et/ou SEQ ID n° 6 et SEQ ID n° 8, SEQ ID n° 10, SEQ ID n° 12 pour la fabrication d'une préparation pharmaceutique destinée au traitement d'une maladie qui est **caractérisée par** une synthèse diminuée d'insuline ou s'accompagne de celle-ci.

5. Utilisation d'un ou plusieurs acides nucléiques selon SEQ ID n° 3 et/ou 5 et 7 ou 9 et/ou d'un ou plusieurs acides nucléiques codant pour la protéine selon SEQ ID n° 12, pour la fabrication d'une préparation pharmaceutique destinée au traitement d'une maladie qui est **caractérisée par** une synthèse diminuée d'insuline ou s'accompagne de celle-ci.

6. Utilisation selon la revendication 4 ou 5, **caractérisée en ce que** la maladie est le diabète.


### Sequenzprotokoll

<110> Ernst-Moritz-Arndt Universität Greifswald
<120> Modulation der Insulinsynthese
<130> P 64322
<160> 12
<170> Patent In version 3.1
<210> 1
   <211> 852
   <212> DNA
   <213> Homo sapiens
<220>
   <221> Stopcodon
   <222> (850)..(852)
   <223> Nukleotidsequenz von PDX-1
<400> 1
   atgaacggcg aggagcagta ctacgcggcc acgcagcttt acaaggaccc atgcgcgttc      60
   cagcgaggcc cggcgccgga gttcagcgcc agcccccctg cgtgcctgta catgggccgc     120
   cagcccccgc cgccgccgcc gcacccgttc cctggcgccc tgggcgcgct ggagcagggc   180
   agccccccgg acatctcccc gtacgaggtg ccccccctcg ccgacgaccc cgcggtggcg   240
   caccttcacc accacctccc ggctcagctc gcgctccccc acccgcccgc cgggcccttc     300
   ccggagggag ccgagccggg cgtcctggag gagcccaacc gcgtccagct gcctttccca     360
   tggatgaagt ctaccaaagc tcacgcgtgg aaaggccagt gggcaggcgg cgcctacgct     420
   gcggagccgg aggagaacaa gcggacgcgc acggcctaca cgcgcgcaca gctgctagag     480
   ctggagaagg agttcctatt caacaagtac atctcacggc cgcgccgggt ggagctggct     540
   gtcatgttga acttgaccga gagacacatc aagatctggt tccaaaaccg ccgcatgaag     600
   tggaaaaagg aggaggacaa gaagcgcggc ggcgggacag ctgtcggggg tggcggggtc     660
   gcggagcctg agcaggactg cgccgtgacc tccggcgagg agcttctggc gctgccgccg     720
   ccgccgcccc ccggaggtgc tgtgccgccc gctgcccccg ttgccgcccg agagggccgc     780
   ctgccgcctg gccttagcgc gtcgccacag ccctccagcg tcgcgcctcg gcggccgcag     840
   gaaccacgat ga                                                         852
<210> 2
   <211> 283
   <212> PRT
   <213> Homo sapiens.
<223> Aminosäuresequenz von PDX-1
<400> 2
<210> 3
   <211> 1182
   <212> DNA
   <213> Homo sapiens
<220>
   <221> Stopcodon
   <222> (1080)..(1082)
   <223> Nukleotidsequenz von CKII-Untereinheit alpha
<400> 3
   atgtcgggac ccgtgccaag cagggccaga gtttacacag atgttaatac acacagacct    60
   cgagaatact gggattacga gtcacatgtg gtggaatggg gaaatcaaga tgactaccag   120
   ctggttcgaa aattaggccg aggtaaatac agtgaagtat ttgaagccat caacatcaca   180
   aataatgaaa aagttgttgt taaaattctc aagccagtaa aaaagaagaa aattaagcgt   240
   gaaataaaga ttttggagaa tttgagagga ggtcccaaca tcatcacact ggcagacatt   300
   gtaaaagacc ctgtgtcacg aacccccgcc ttggtttttg aacacgtaaa caacacagac    360
   ttcaagcaat tgtaccagac gttaacagac tatgatattc gattttacat gtatgagatt   420
   ctgaaggccc tggattattg tcacagcatg ggaattatgc acagagatgt caagccccat   480
   aatgtcatga ttgatcatga gcacagaaag ctacgactaa tagactgggg tttggctgag   540
   ttttatcatc ctggccaaga atataatgtc cgagttgctt cccgatactt caaaggtcct   600
   gagctacttg tagactatca gatgtacgat tatagtttgg atatgtggag tttgggttgt   660
   atgctggcaa gtatgatctt tcggaaggag ccatttttcc atggacatga caattatgat   720
   cagttggtga ggatagccaa ggttctgggg acagaagatt tatatgacta tattgacaaa   780
   tacaacattg aattagatcc acgtttcaat gatatcttgg gcagacactc tcgaaagcga   840
   tgggaacgct ttgtccacag tgaaaatcag caccttgtca gccctgaggc cttggatttc   900
   ctggacaaac tgctgcgata tgaccaccag tcacggctta ctgcaagaga ggcaatggag   960
   cacccctatt tctacactgt tgtgaaggac caggctcgaa tgggttcatc tagcatgcca  1020
   gggggcagta cgcccgtcag cagcgccaat atgatgtcag ggatttcttc agtgccaacc  1080
   ccttcacccc ttggacctct ggcaggctca ccagtgattg ctgctgccaa cccccttggg   1140
   atgcctgttc cagctgccgc tggcgctcag cagtaacggc cc                     1182
<210> 4
   <211> 391
   <212> PRT
   <213> Homo sapiens
<223> Aminosäuresequenz von CKII-Untereinheit alpha
<400> 4
<210> 5
   <211> 1053
   <212> DNA
   <213> Homo sapiens
<220>
   <221> Stopcodon
   <222> (1051)..(1053)
   <223> Nukleotidsequenz von CKII-Untereinheit alpha'
<400> 5
   atgcccggcc cggccgcggg cagcagggcc cgggtctacg ccgaggtgaa cagtctgagg     60
   agccgcgagt actgggacta cgaggctcac gtcccgagct ggggtaatca agatgattac    120
   caactggttc gaaaacttgg tcggggaaaa tatagtgaag tatttgaggc cattaatatc    180
   accaacaatg agagagtggt tgtaaaaatc ctgaagccag tgaagaaaaa gaagataaaa    240
   cgagaggtta agattctgga gaaccttcgt ggtggaacaa atatcattaa gctgattgac    300
   actgtaaagg accccgtgtc aaagacacca gctttggtat ttgaatatat caataataca    360
   gattttaagc aactctacca gatcctgaca gactttgata tccggtttta tatgtatgaa    420
   ctacttaaag ctctggatta ctgccacagc aagggaatca tgcacaggga tgtgaaacct    480
   cacaatgtca tgatagatca ccaacagaaa aagctgcgac tgatagattg gggtctggca    540
   gaattctatc atcctgctca ggagtacaat gttcgtgtag cctcaaggta cttcaaggga    600
   ccagagctcc tcgtggacta tcagatgtat gattatagct tggacatgtg gagtttgggc    660
   tgtatgttag caagcatgat ctttcgaagg gaaccattct tccatggaca ggacaactat    720
   gaccagcttg ttcgcattgc caaggttctg ggtacagaag aactgtatgg gtatctgaag    780
   aagtatcaca tagacctaga tccacacttc aacgatatcc tgggacaaca ttcacggaaa    840
   cgctgggaaa actttatcca tagtgagaac agacaccttg tcagccctga ggccctagat    900
   cttctggaca aacttctgcg atacgaccat caacagagac tgactgccaa agaggccatg    960
   gagcacccat acttctaccc tgtggtgaag gagcagtccc agccttgtgc agacaatgct   1020
   gtgctttcca gtggtctcac ggcagcacga tga                                1053
<210> 6
   <211> 350
   <212> PRT
   <213> Homo sapiens
<223> Aminosäuresequenz von CKII-Untereinheit alpha'
<400> 6
   Met Pro Gly Pro Ala Ala Gly Ser Arg Ala Arg Val Tyr Ala Glu Val
   1               5                   10                  15
   Asn Ser Leu Arg Ser Arg Glu Tyr Trp Asp Tyr Glu Ala His Val Pro
               20                  25                  30
   Ser Trp Gly Asn Gln Asp Asp Tyr Gln Leu Val Arg Lys Leu Gly Arg
           35                  40                  45
   Gly Lys Tyr Ser Glu Val Phe Glu Ala Ile Asn Ile Thr Asn Asn Glu
       50                  55                  60
   Arg Val Val Val Lys Ile Leu Lys Pro Val Lys Lys Lys Lys Ile Lys
   65                  70                  75                  80
   Arg Glu Val Lys Ile Leu Glu Asn Leu Arg Gly Gly Thr Asn Ile Ile
                   85                  90                  95
   Lys Leu Ile Asp Thr Val Lys Asp Pro Val Ser Lys Thr Pro Ala Leu
               100                 105                 110
   Val Phe Glu Tyr Ile Asn Asn Thr Asp Phe Lys Gln Leu Tyr Gln Ile
           115                 120                 125
   Leu Thr Asp Phe Asp Ile Arg Phe Tyr Met Tyr Glu Leu Leu Lys Ala
       130                 135                 140
   Leu Asp Tyr Cys His Ser Lys Gly Ile Met His Arg Asp Val Lys Pro
   145                 150                 155                 160
   His Asn Val Met Ile Asp His Gln Gln Lys Lys Leu Arg Leu Ile Asp
                   165                 170                 175
   Trp Gly Leu Ala Glu Phe Tyr His Pro Ala Gln Glu Tyr Asn Val Arg
               180                 185                 190
   Val Ala Ser Arg Tyr Phe Lys Gly Pro Glu Leu Leu Val Asp Tyr Gln
           195                 200                 205
   Met Tyr Asp Tyr Ser Leu Asp Met Trp Ser Leu Gly Cys Met Leu Ala
       210                 215                 220
   Ser Met Ile Phe Arg Arg Glu Pro Phe Phe His Gly Gln Asp Asn Tyr
   225                 230                 235                 240
   Asp Gln Leu Val Arg Ile Ala Lys Val Leu Gly Thr Glu Glu Leu Tyr
                   245                 250                 255
   Gly Tyr Leu Lys Lys Tyr His Ile Asp Leu Asp Pro His Phe Asn Asp
               260                 265                 270
   Ile Leu Gly Gln His Ser Arg Lys Arg Trp Glu Asn Phe Ile His Ser
   275 280 285
   Glu Asn Arg His Leu Val Ser Pro Glu Ala Leu Asp Leu Leu Asp Lys
   290 295 300
   Leu Leu Arg Tyr Asp His Gln Gln Arg Leu Thr Ala Lys Glu Ala Met
   305 310 315 320
   Glu His Pro Tyr Phe Tyr Pro Val Val Lys Glu Gln Ser Gln Pro Cys
   325 330 335
   Ala Asp Asn Ala Val Leu Ser Ser Gly Leu Thr Ala Ala Arg
   340 345 350
<210> 7
   <211> 648
   <212> DNA
   <213> Homo sapiens
<220>
   <221> Stopcodon
   <222> (646) .. (648)
   <223> Nukleotidsequenz von CKII-Untereinheit beta
<400> 7
   atgagcagct cagaggaggt gtcctggatt tcctggttct gtgggctccg tggcaatgaa 60
   ttcttctgtg aagtggatga agactacatc caggacaaat ttaatcttac tggactcaat 120
   gagcaggtcc ctcactatcg acaagctcta gacatgatct tggacctgga gcctgatgaa 180
   gaactggaag acaaccccaa ccagagtgac ctgattgagc aggcagccga gatgctttat 240
   ggattgatcc acgcccgcta catccttacc aaccgtggca tcgcccagat gttggaaaag 300
   taccagcaag gagactttgg ttactgtcct cgtgtgtact gtgagaacca gccaatgctt 360
   cccattggcc tttcagacat cccaggtgaa gccatggtga agctctactg ccccaagtgc 420
   atggatgtgt acacacccaa gtcatcaaga caccatcaca cggatggcgc ctacttcggc 480
   actggtttcc ctcacatgct cttcatggtg catcccgagt accggcccaa gagacctgcc 540
   aaccagtttg tgcccaggct ctacggtttc aagatccatc cgatggccta ccagctgcag 600
   ctccaagccg ccagcaactt caagagccca gtcaagacga ttcgctga 648
<210> 8
   <211> 215
   <212> PRT
   <213> Homo sapiens.
<223> Aminosäuresequenz von CKII-Untereinheit beta
<400> 8
   Met Ser Ser Ser Glu Glu Val Ser Trp Ile Ser Trp Phe Cys Gly Leu
   1               5                   10                  15
   Arg Gly Asn Glu Phe Phe Cys Glu Val Asp Glu Asp Tyr Ile Gln Asp
               20                  25                  30
   Lys Phe Asn Leu Thr Gly Leu Asn Glu Gln Val Pro His Tyr Arg Gln
           35                  40                  45
   Ala Leu Asp Met Ile Leu Asp Leu Glu Pro Asp Glu Glu Leu Glu Asp
       50                  55                  60
   Asn Pro Asn Gln Ser Asp Leu Ile Glu Gln Ala Ala Glu Met Leu Tyr
   65                  70                  75                  80
   Gly Leu Ile His Ala Arg Tyr Ile Leu Thr Asn Arg Gly Ile Ala Gln
                   85                  90                  95
   Met Leu Glu Lys Tyr Gln Gln Gly Asp Phe Gly Tyr Cys Pro Arg Val
               100                 105                 110
   Tyr Cys Glu Asn Gln Pro Met Leu Pro Ile Gly Leu Ser Asp Ile Pro
           115                 120                 125
   Gly Glu Ala Met Val Lys Leu Tyr Cys Pro Lys Cys Met Asp Val Tyr
       130                 135                 140
   Thr Pro Lys Ser Ser Arg His His His Thr Asp Gly Ala Tyr Phe Gly
   145                 150                 155                 160
   Thr Gly Phe Pro His Met Leu Phe Met Val His Pro Glu Tyr Arg Pro
                   165                 170                 175
   Lys Arg Pro Ala Asn Gln Phe Val Pro Arg Leu Tyr Gly Phe Lys Ile
               180                 185                 190
   His Pro Met Ala Tyr Gln Leu Gln Leu Gln Ala Ala Ser Asn Phe Lys
           195                 200                 205
   Ser Pro Val Lys Thr Ile Arg
   210 215
<210> 9
   <211> 768
   <212> DNA
   <213> Homo sapiens
<220>
   <221> Stopcodon
   <222> (766)..(768)
   <223> Nukleotidsequenz von 14-3-3 epsilon
<400> 9
   atggatgatc gagaggatct ggtgtaccag gcgaagctgg ccgagcaggc tgagcgatac      60
   gacgaaatgg tggagtcaat gaagaaagta gcagggatgg atgtggagct gacagttgaa     120
   gaaagaaacc tcctatctgt tgcatataag aatgtgattg gagctagaag agcctcctgg     180
   agaataatca gcagcattga acagaaagaa gaaaacaagg gaggagaaga caagctaaaa     240
   atgattcggg aatatcggca aatggttgag actgagctaa agttaatctg ttgtgacatt     300
   ctggatgtac tggacaaaca cctcattcca gcagctaaca ctggcgagtc caaggttttc     360
   tattataaaa tgaaagggga ctaccacagg tatctggcag aatttgccac aggaaacgac     420
   aggaaggagg ctgcggagaa cagcctagtg gcttataaag ctgctagtga tattgcaatg     480
   acagaacttc caccaacgca tcctattcgc ttaggtcttg ctctcaattt ttccgtattc     540
   tactacgaaa ttcttaattc ccctgaccgt gcctgcaggt tggcaaaagc agcttttgat     600
   gatgcaattg cagaactgga tacgctgagt gaagaaagct ataaggactc tacacttatc     660
   atgcagttgt tacgtgataa tctgacacta tggacttcag acatgcaggg tgacggtgaa     720
   gagcagaata aagaagcgct gcaggacgtg gaagacgaaa atcagtga                  768
<210> 10
   <211> 255
   <212> PRT
   <213> Homo sapiens
<223> Aminosäuresequenz von 14-3-3 epsilon
<400> 10
   Met Asp Asp Arg Glu Asp Leu Val Tyr Gln Ala Lys Leu Ala Glu Gln
   1 5 10 15
   Ala Glu Arg Tyr Asp Glu Met Val Glu Ser Met Lys Lys Val Ala Gly
   20 25 30
   Met Asp Val Glu Leu Thr Val Glu Glu Arg Asn Leu Leu Ser Val Ala
   35 40 45
   Tyr Lys Asn Val Ile Gly Ala Arg Arg Ala Ser Trp Arg Ile Ile Ser
   50 55 60
   Ser Ile Glu Gln Lys Glu Glu Asn Lys Gly Gly Glu Asp Lys Leu Lys
   65 70 75 80
   Met Ile Arg Glu Tyr Arg Gln Met Val Glu Thr Glu Leu Lys Leu Ile
   85 90 95
   Cys Cys Asp Ile Leu Asp Val Leu Asp Lys His Leu Ile Pro Ala Ala
   100 105 110
   Asn Thr Gly Glu Ser Lys Val Phe Tyr Tyr Lys Met Lys Gly Asp Tyr
   115 120 125
   His Arg Tyr Leu Ala Glu Phe Ala Thr Gly Asn Asp Arg Lys Glu Ala
   130 135 140
   Ala Glu Asn Ser Leu Val Ala Tyr Lys Ala Ala Ser Asp Ile Ala Met
   145 150 155 160
   Thr Glu Leu Pro Pro Thr His Pro Ile Arg Leu Gly Leu Ala Leu Asn
   165 170 175
   Phe Ser Val Phe Tyr Tyr Glu Ile Leu Asn Ser Pro Asp Arg Ala Cys
   180 185 190
   Arg Leu Ala Lys Ala Ala Phe Asp Asp Ala Ile Ala Glu Leu Asp Thr
   195 200 205
   Leu Ser Glu Glu Ser Tyr Lys Asp Ser Thr Leu Ile Met Gln Leu Leu
   210 215 220
   Arg Asp Asn Leu Thr Leu Trp Thr Ser Asp Met Gln Gly Asp Gly Glu
   225 230 235 240
   Glu Gln Asn Lys Glu Ala Leu Gln Asp Val Glu Asp Glu Asn Gln
   245 250 255
<210> 11
   <211> 1284
   <212> DNA
   <213> Homo sapiens.
<220>
   <221> Stopcodon
   <222> (1282)..(1284)
   <223> Nukleotidsequenz der kurzen Isoform von EED
<400> 11
   atgcctgcgg ccaagaagca gaagctgagc agtgacgaga acagcaatcc agaactctct     60
   ggagacgaga atgatgacgc tgtcagtata gaaagtggta caaacactga acgccctgat    120
   acacctacaa acacgccaaa tgcacctgga aggaaaagtt ggggaaaggg aaaatggaag    180
   tcaaagaaat gcaaatattc tttcaaatgt gtaaatagtc tcaaggaaga tcataaccaa    240
   ccattgtttg gagttcagtt taactggcac agtaaagaag gagatccatt agtgtttgca    300
   actgtaggaa gcaacagagt taccttgtat gaatgtcatt cacaaggaga aatccggttg    360
   ttgcaatctt acgtggatgc tgatgctgat gaaaactttt acacttgtgc atggacctat    420
   gatagcaata cgagccatcc tctgctggct gtagctggat ctagaggcat aattaggata    480
   ataaatccta taacaatgca gtgtataaag cactatgttg gccatggaaa tgctatcaat    540
   gagctgaaat tccatccaag agatccaaat cttctcctgt cagtaagtaa agatcatgct    600
   ttacgattat ggaatatcca gacggacact ctggtggcaa tatttggagg cgtagaaggg    660
   cacagagatg aagttctaag tgctgattat gatcttttgg gtgaaaaaat aatgtcctgt    720
   ggtatggatc attctcttaa actttggagg atcaattcaa agagaatgat gaatgcaatt    780
   aaggaatctt atgattataa tccaaataaa actaacaggc catttatttc tcagaaaatc    840
   cattttcctg atttttctac cagagacata cataggaatt atgttgattg tgtgcgatgg    900
   ttaggcgatt tgatactttc taagtcttgt gaaaatgcca ttgtgtgctg gaaacctggc    960
   aagatggaag atgatataga taaaattaaa cccagtgaat ctaatgtgac tattcttggg   1020
   cgatttgatt acagccagtg tgacatttgg tacatgaggt tttctatgga tttctggcaa   1080
   aagatgcttg cattgggcaa tcaagttggc aaactttatg tttgggattt agaagtagaa   1140
   gatcctcata aagccaaatg tacaacactg actcatcata aatgtggtgc tgctattcga   1200
   caaaccagtt ttagcaggga tagcagcatt cttatagctg tttgtgatga tgccagtatt   1260
   tggcgctggg atcgacttcg ataa                                          1284
<210> 12
   <211> 427
   <212> PRT
   <213> Homo sapiens.
<223> Aminosäuresequenz der kurzen Isoform von EED
<400> 12
   Met Pro Ala Ala Lys Lys Gln Lys Leu Ser Ser Asp Glu Asn Ser Asn
   1 5 10 15
   Pro Glu Leu Ser Gly Asp Glu Asn Asp Asp Ala Val Ser Ile Glu Ser
   20 25 30
   Gly Thr Asn Thr Glu Arg Pro Asp Thr Pro Thr Asn Thr Pro Asn Ala
   35 40 45
   Pro Gly Arg Lys Ser Trp Gly Lys Gly Lys Trp Lys Ser Lys Lys Cys
   50 55 60
   Lys Tyr Ser Phe Lys Cys Val Asn Ser Leu Lys Glu Asp His Asn Gln
   65 70 75 80
   Pro Leu Phe Gly Val Gln Phe Asn Trp His Ser Lys Glu Gly Asp Pro
   85 90 95
   Leu Val Phe Ala Thr Val Gly Ser Asn Arg Val Thr Leu Tyr Glu Cys
   100 105 110
   His Ser Gln Gly Glu Ile Arg Leu Leu Gln Ser Tyr Val Asp Ala Asp
   115 120 125
   Ala Asp Glu Asn Phe Tyr Thr Cys Ala Trp Thr Tyr Asp Ser Asn Thr
   130 135 140
   Ser His Pro Leu Leu Ala Val Ala Gly Ser Arg Gly Ile Ile Arg Ile
   145 150 155 160
   Ile Asn Pro Ile Thr Met Gln Cys Ile Lys His Tyr Val Gly His Gly
   165 170 175
   Asn Ala Ile Asn Glu Leu Lys Phe His Pro Arg Asp Pro Asn Leu Leu
   180 185 190
   Leu Ser Val Ser Lys Asp His Ala Leu Arg Leu Trp Asn Ile Gln Thr
   195 200 205
   Asp Thr Leu Val Ala Ile Phe Gly Gly Val Glu Gly His Arg Asp Glu
   210 215 220
   Val Leu Ser Ala Asp Tyr Asp Leu Leu Gly Glu Lys Ile Met Ser Cys
   225 230 235 240
   Gly Met Asp His Ser Leu Lys Leu Trp Arg Ile Asn Ser Lys Arg Met
   245 250 255
   Met Asn Ala Ile Lys Glu Ser Tyr Asp Tyr Asn Pro Asn Lys Thr Asn
   260 265 270
   Arg Pro Phe Ile Ser Gln Lys Ile His Phe Pro Asp Phe Ser Thr Arg
   275 280 285
   Asp Ile His Arg Asn Tyr Val Asp Cys Val Arg Trp Leu Gly Asp Leu
   290 295 300
   Ile Leu Ser Lys Ser Cys Glu Asn Ala Ile Val Cys Trp Lys Pro Gly
   305 310 315 320
   Lys Met Glu Asp Asp Ile Asp Lys Ile Lys Pro Ser Glu Ser Asn Val
   325 330 335
   Thr Ile Leu Gly Arg Phe Asp Tyr Ser Gln Cys Asp Ile Trp Tyr Met
   340 345 350
   Arg Phe Ser Met Asp Phe Trp Gln Lys Met Leu Ala Leu Gly Asn Gln
   355 360 365
   Val Gly Lys Leu Tyr Val Trp Asp Leu Glu Val Glu Asp Pro His Lys
   370 375 380
   Ala Lys Cys Thr Thr Leu Thr His His Lys Cys Gly Ala Ala Ile Arg
   385 390 395 400
   Gln Thr Ser Phe Ser Arg Asp Ser Ser Ile Leu Ile Ala Val Cys Asp
   405 410 415
   Asp Ala Ser Ile Trp Arg Trp Asp Arg Leu Arg
   420 425